# EUROPÄISCHE PATENTSCHRIFT

(11) **EP 1 431 272 B1**
(45) Veröffentlichungstag und Bekanntmachung des Hinweises auf die Patenterteilung: **14.05.2008**
(21) Anmeldenummer: 03026512.8
(22) Anmeldetag: 18.11.2003
(51) Int. Cl.: C07C 209/26

(54) **Verfahren zur katalytischen Hydrierung einer aliphatisch ungesättigten Gruppe in einer organischen Verbindung**
Process for the catalysed hydrogenation of an aliphatic unsaturated group into an organic compound
Procédé d'hydrogénation catalysé d'un groupe aliphatique insaturé en un composé organique

(30) Priorität: 20.12.2002 DE 10261194
(43) Veröffentlichungstag der Anmeldung: 23.06.2004
(73) Patentinhaber: BASF SE, 67056 Ludwigshafen (DE)
(72) Erfinder: Gerlach, Till, Dr., 67071 Ludwigshafen (DE); Funke, Frank, Dr., 67067 Ludwigshafen (DE); Melder, Johann-Peter, Dr., 67459 Böhl-Iggelheim (DE)

(56) Entgegenhaltungen:
- EP-A- 0 394 841
- EP-A- 0 394 842
- EP-A- 0 849 841

## Beschreibung

Die vorliegende Erfindung betrifft ein Verfahren zur katalytischen Hydrierung einer aliphatisch ungesättigten Gruppe in einer organischen Verbindung.

EP-A1-394 841 und EP-A1-394 842 (beide BASF AG) beschreiben die Verwendung von Zirkoniumdioxid, Kupfer und Nickel enthaltenden Katalysatoren in Verfahren zur Hydrierung von aliphatischen CC-Doppel- und CC-Dreifachbindungen.

EP-A1-742 045 (BASF AG) betrifft bestimmte Kobaltkatalysatoren und ihre Verwendung in Verfahren zur Hydrierung von organischen Nitrilen und Iminen.

Die Zirkoniumoxid-haltigen Katalysatoren des Stands der Technik sind bevorzugt durch gemeinsame Fällung von wasserlöslichen Verbindungen entsprechender Metalle einschließlich des Zirkoniums mittels Mineralbasen und anschließende Trocknung und Temperung erhältlich (Mischfällung; vgl. z.B. EP-A1-394 842, Spalte 2, Zeilen 34 bis 51).

Man kann aber auch in der o.g. Mischfällung einen Teil des wasserlöslichen Zirkoniumsalzes durch festes Zirkoniumdioxid ersetzen (vgl. z.B. EP-A1-394 842, Spalte 2, Zeile 52, bis Spalte 3, Zeile 3).

Eine parallele deutsche Patentanmeldung (BASF AG) mit gleichem Anmeldetag beschreibt ein Verfahren zur Herstellung eines Amins durch Umsetzung eines primären oder sekundären Alkohols, Aldehyds oder Ketons mit Wasserstoff und einer Stickstoffverbindung, ausgewählt aus der Gruppe Ammoniak, primäre und sekundäre Amine, in Gegenwart eines Katalysators, wobei man einen Katalysator einsetzt, bei dessen Herstellung eine Fällung von katalytisch aktiven Komponenten auf monoklines, tetragonales oder kubisches Zirkoniumdioxid erfolgte.

Eine parallele deutsche Patentanmeldung (BASF AG) mit gleichem Anmeldetag betrifft ein Verfahren zur Herstellung eines symmetrischen sekundären Amins durch Umsetzung eines primären Amins in Gegenwart von Wasserstoff und eines Katalysators, dadurch gekennzeichnet, dass man einen Katalysator einsetzt, bei dessen Herstellung eine Fällung von katalytisch aktiven Komponenten auf monoklines, tetragonales oder kubisches Zirkoniumdioxid erfolgte.

Nachteilig an den Zirkoniumdioxid-haltigen Heterogenkatalysatoren des Stands der Technik für Hydrierungen von aliphatisch ungesättigten Gruppen in organischen Verbindungen ist deren erfindungsgemäß erkannte Abnahme der mechanischen Stabilität unter den Reaktionsbedingungen ihres Einsatzes, insbesondere in Gegenwart von Reaktionsmedien, die Wasser enthalten. Eine Folge von mechanisch weniger stabilen Heterogenkatalysatoren ist der häufiger notwendige Katalysatorwechsel im Reaktor und damit eine erniedrigte Raum-Zeit-Ausbeute.

Der vorliegenden Erfindung lag demgemäß die Aufgabe zugrunde, den Nachteilen des Stands der Technik abzuhelfen und ein verbessertes Verfahren zur katalytischen Hydrierung einer aliphatisch ungesättigten Gruppe in organischen Verbindungen bereitzustellen, wobei ein Katalysator mit verbesserten mechanischen Eigenschaften unter den Reaktionsbedingungen seines Einsatzes verwendet wird und damit die Wirtschaftlichkeit bisheriger Verfahren, insbesondere solchen, in denen Zirkoniumdioxid-haltige Katalysatoren zum Einsatz kommen, zu verbessern.

Erfindungsgemäß wurde erkannt, dass ein Grund für die verbesserungswürdige mechanische Stabilität, d.h. die mechanische Erweichung, der bekannten Zirkoniumdioxid-haltigen Katalysatoren unter Reaktionsbedingungen die Tatsache ist, dass das Zirkoniumdioxid, z.B. bei Anwendung der o.g. (Misch)Fäll-Technik, zunächst ganz oder teilweise amorph vorliegt und unter den Bedingungen der mit diesen Katalysatoren katalysierten chemischen Reaktion ganz oder teilweise eine Kristallisation, also Umwandlung der Modifikation, zu tetragonalem, monoklinem oder kubischem Zirkoniumdioxid stattfindet. Bei Hydrierreaktionen werden als Reaktionsbedingungen zumeist eine erhöhte Temperatur (z.B. 40 - 300 °C) und erhöhter Druck (z.B. 5 - 320 bar) angewendet.

Demgemäß wurde gefunden, dass durch die Verwendung von Zirkoniumdioxid, das eine-unter den Reaktionsbedingungen der Hydrierreaktion thermodynamisch stabile oder zumindest metastabile Modifikation aufweist, wie monoklines, tetragonales oder kubisches Zirkoniumdioxid, bei der Herstellung eines Zirkoniumdioxid-haltigen Katalysators die mechanische Stabilität der resultierenden Katalysatoren unter den Reaktionsbedingungen, insbesondere in Gegenwart von entsprechenden Reaktionsmedien, die Wasser enthalten, wesentlich erhöht wird.

Gegenstand der Erfindung ist demnach ein Verfahren zur katalytischen Hydrierung einer aliphatisch ungesättigten Gruppe in einer organischen Verbindung, welches dadurch gekennzeichnet ist, dass man einen Katalysator einsetzt, bei dessen Herstellung eine Fällung von katalytisch aktiven Komponenten auf monoklines, tetragonales oder kubisches Zirkoniumdioxid erfolgte,
und dass die katalytisch aktive Masse des Katalysators vor der Behandlung mit Wasserstoff
20 bis 85 Gew.-% sauerstoffhaltige Verbindungen des Zirkoniums, berechnet als ZrO₂, 1 bis 30 Gew.-% sauerstoffhaltige Verbindungen des Kupfers, berechnet als CuO, und 14 bis 70 Gew.-% sauerstoffhaltige Verbindungen des Nickels, berechnet als NiO, enthält.

Bei den aufgefällten katalytisch aktiven Komponenten handelt es sich insbesondere um Metallsalze, wobei das Metall aus den Gruppen 8 bis 11 (entsprechend den Nebengruppen VIII und IB) des Periodensystems der Elemente, ganz besonders aus der Gruppe Fe, Co, Ni, Ru, Rh, Pd, Pt und Cu, ausgewählt ist.

Besonders bevorzugt ist das Metall aus der Gruppe Cu, Ni und Co ausgewählt.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z. B. Stränge) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators vor der Behandlung mit Wasserstoff.

Die katalytisch aktive Masse des Katalysators ist als die Summe der Massen der katalytisch aktiven Bestandteile definiert und enthält, vor der Behandlung mit Wasserstoff, im wesentlichen die katalytisch aktiven Bestandteile monoklines, tetragonales oder kubisches Zirkoniumdioxid (oder Mischungen dieser Modifikationen) und Metallsalze als weitere katalytisch aktive Komponenten.

Die Summe der o.g. katalytisch aktiven Bestandteile, berechnet in oxidischer Form, z.B. als ZrO₂, CuO, NiO und CoO, in der katalytisch aktive Masse vor der Behandlung mit Wasserstoff beträgt im allgemeinen 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt >98 bis 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen IA bis VIA und IB bis VIIB und VIII des Periodensystems, enthalten.

Beispiele für solche Elemente bzw. deren Verbindungen sind:
Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Silber bzw. Silberoxide, Lanthanide, wie Ce bzw. CeO₂ oder Pr bzw. Pr₂O₃, Alkalimetalloxide, wie Na₂O, Alkalimetallcarbonate, wie Na₂CO₃ und K₂CO₃, Erdalkalimetalloxide, wie SrO, Erdalkalimetallcarbonate, wie MgCO₃, CaCO₃, BaCO₃, Phosphorsäureanhydride und Boroxid (B₂O₃).

Bevorzugte im erfindungsgemäßen Verfahren verwendete Katalysatoren enthalten in ihrer katalytisch aktiven Masse vor der Behandlung mit Wasserstoff
20 bis 65 Gew.-%, besonders bevorzugt 22 bis 40 Gew.-%, monoklines, tetragonales oder kubisches Zirkoniumdioxid (ZrO₂) (oder Mischungen dieser Modifikationen),
1 bis 30 Gew.-%, besonders bevorzugt 2 bis 25 Gew.-%, sauerstoffhaltige Verbindungen des Kupfers, berechnet als CuO, und
15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist,
Besonders bevorzugte Katalysatoren enthalten zusätzlich in ihrer katalytisch aktiven Masse vor der Behandlung mit Wasserstoff
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Die erfindungsgemäß eingesetzten Katalysatoren lassen sich wie folgt herstellen.

Unter Auffällung wird eine Katalysatorherstellung verstanden, bei der ein schwer lösliches Trägermaterial in einer Flüssigkeit, meist Wasser, suspendiert wird, die Dotierungskomponenten, als leicht lösliche Verbindungen eingesetzt, in einer Flüssigkeit, meist Wasser, gelöst und dann durch Zugabe eines Fällungsmittels auf den suspendierten Träger ausgefällt werden (z.B. beschrieben in EP-A1-394 842, Spalten 2-3; EP-A2-394 841, Seite 2, Zeile 45, bis Seite 3, Zeile 22; und A. B. Stiles, Catalyst Manufacture, Marcel Dekker, Inc., 1983, Seite 15).

Gemäß der vorliegenden Erfindung wird zur Herstellung des Katalysators als Trägermaterial Zirkoniumdioxid in einer thermodynamisch stabilen oder metastabilen Modifikation, d.h. in der monoklinen, tetragonalen oder kubischen Modifikation, eingesetzt.

Die grundlegenden Eigenschaften von Zirkoniumdioxid sind in K. Dyrek, A. Adamski, Z. Sojka, Ceramic Interfaces 2, University Press, Cambridge, 2001, S. 241 - 259, zusammengefasst, so etwa die existierenden Modifikationen monoklin, tetragonal und kubisch und deren Herstellung.

Die Zirkoniumdioxid-Kristallstruktur kann, insbesondere bei der tetragonalen Modifikation, durch Zusätze von einem oder mehreren Metalloxiden der Nebengruppe IIIB oder der Hauptgruppe IIA des Periodensystems, insbesondere Yttriumoxid, Calciumoxid, Lanthanoxid, Magnesiumoxid oder Scandiumoxid, weiter stabilisiert sein.

Diese Stabilisierung bewirkt z.B. ganz oder teilweise eine Hemmung der Umwandlung der tetragonalen Modifikation in die thermodynamisch stabilste monokline Modifikation.

Zur Herstellung des Katalysators wird das Zirkoniumdioxid in einem Lösungsmittel, z.B. in Wasser, suspendiert. Dann werden die z.B. in Wasser gelösten Metallsalze zugegeben und anschließend durch Zugabe von z.B. Lauge in Form von im verwendeten Lösungsmittel, z.B. Wasser, schwer- oder unlöslichen, basischen Salzen gefällt.

Die bei diesen Fällungsreaktionen erhaltenen Niederschläge sind im allgemeinen chemisch uneinheitlich und enthalten in der Regel Mischungen der Oxide, Oxidhydrate, Hydroxide, Carbonate und/oder Hydrogencarbonate der eingesetzten Metalle.

Die Fällung kann z.B. bei Temperaturen von 20 - 100 °C, besonders 50 - 90 °C, insbesondere bei 60 - 80 °C, durchgeführt werden.

Alternativ kann auch die Metallsalzlösung und die Lauge gleichzeitig in einen Kessel geleitet werden, der die Zirkoniumdioxid-Trägersuspension enthält. Der Träger kann auch in der Metallsalzlösung suspendiert werden und diese dann gleichzeitig mit der Lauge in einen Fällkessel geleitet werden.

Die weitere Katalysatorherstellung wird dann nach bekannten Methoden, also etwa Filtration, Waschung, Trocknung, Calcinierung, Formgebung, Reduktion/Passivierung durchgeführt.

Die auf diese Weise hergestellten Katalysatoren enthalten vor der Reduktion/Passivierung die katalytisch aktiven Metalle in Form eines Gemisches ihrer sauerstoffhaltigen Verbindungen, d.h. insbesondere als Oxide und Mischoxide.

Die hergestellten Katalysatoren können als solche gelagert werden. Vor ihrem Einsatz als Katalysatoren zur Hydrierung einer aliphatisch ungesättigten Gruppe in einer organischen Verbindung werden sie üblicherweise durch Behandlung mit Wasserstoff vorreduziert. Sie können jedoch auch ohne Vorreduktion eingesetzt werden, wobei sie dann unter den Bedingungen der Hydrierreaktion durch den im Reaktor vorhandenen Wasserstoff reduziert werden. Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Bei der aliphatisch ungesättigten Gruppe in den im erfindungsgemäßen Verfahren eingesetzten organischen Verbindungen handelt es sich insbesondere um aliphatische CC-Doppel- oder CN-Doppelbindungen, aliphatischen CC-Dreifach- oder CN-Dreifachbindungen, oder Aldehyd- oder Ketogruppen.

Z.B. ist die Hydrierung von aliphatisch ungesättigten Verbindungen der Formel I 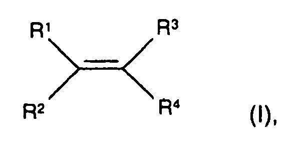 und der Formel III 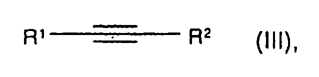 in denen
- R¹, R², R³, R⁴: Wasserstoff (H), Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, oder Alkylheteroaryl, wie C₄₋₂₀-Alkylheteroaryl,
bedeuten,
und im Fall von ungesättigten Verbindungen der Formel I R¹ und R² auch gemeinsam (CH₂)ₗCH₂-X-(CH₂)ₘ, oder R² und R⁴ auch gemeinsam (CH₂)ₗ-X-(CH₂)ₘ, mit
- X: CH₂, CHR⁵, Sauerstoff (O), Schwefel (S) oder NR⁵,
- R⁵: Wasserstoff (H), Alkyl, wie C₁₋₄-Alkyl, Alkylphenyl, wie C₇₋₄₀-Alkylphenyl,
- l, m: eine ganze Zahl von 1 bis 4,
bedeuten können, wirtschaftlich von besonderem Interesse.

Das erfindungsgemäße Hydrierverfahren findet daher zur Herstellung der Verbindungen II (aus I) und IV und V (aus III), wobei R¹, R², R³ und R⁴ die oben genannten Bedeutungen haben, Anwendung. 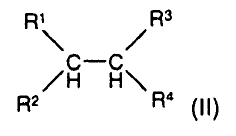 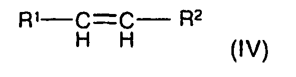 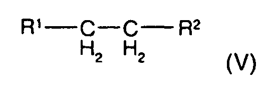 Z.B. ist das erfindungsgemäße Verfahren auch für die Hydrierung von Nitrilen der Formel VI 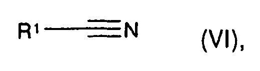 in der
- R¹: Alkyl, wie C₁₋₂₀₀-Alkyl, Cycloalkyl, wie C₃₋₁₂Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, oder Alkylheteroaryl, wie C₄₋₂₀-Alkylheteroaryl,
bedeutet, zu den entsprechenden primären Aminen VII

R¹-CH₂NH₂ (VII)

wirtschaftlich von besonderem Interesse.

Weiterhin ist das erfindungsgemäße Verfahren auch zur Hydrierung von Aldehyden oder Aldiminen der Formel VIIIa bzw. VIIIb

R¹-CHO (VIIIa)

R¹-CH=NH (VIIIb)

in der
- R¹: Wasserstoff (H), Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkylaminoalkyl, Alkoxyalkyl, wie C₂₋₂₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, oder Alkylheteroaryl, wie C₄₋₂₀-Alkylheteroaryl,
bedeutet, zu den entsprechenden primären Alkoholen IXa bzw. primären Aminen IXb

R¹-CH₂OH (IXa),

R¹-CH₂N_{H2} (IXb)

wirtschaftlich von besonderem Interesse.

Zusätzlich ist das erfindungsgemäße Verfahren auch zur Hydrierung von Ketonen oder Ketiminen der Formel Xa bzw. Xb 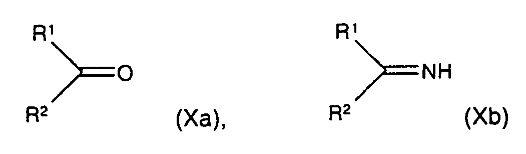 in der
- R¹, R²: Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₂₋₂₀- Hydroxyalkyl-aminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl, oder
- R¹ und R²: gemeinsam (CH₂)ₗ-CH₂-X-(CH₂)ₘ
- X: CH₂, CHR⁵, Sauerstoff (O), Schwefel (S) oder NR⁵,
- R⁵: Wasserstoff (H), Alkyl, wie C₁₋₄-Alkyl, Alkylphenyl, wie C₇₋₄₀-Alkylphenyl, und
- l, m: eine ganze Zahl von 1 bis 4
bedeuten, zu den entsprechenden sekundären Alkoholen XIa bzw. primären Aminen XIb 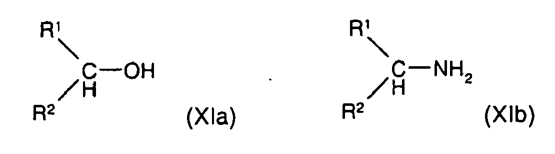 wirtschaftlich von besonderem Interesse.

Die Substituenten R¹ bis R⁵, die Variable X und die Indizes I, m in den Verbindungen I, II, III, IV, V, VI, VII, VIIIa, VIIIb, IXa, IXb, Xa, Xb, XIa und XIb haben unabhängig voneinander folgende Bedeutungen:
R¹, R², R³, R⁴:
   - Alkyl, wie C₁₋₂₀₀-Alkyl, bevorzugt C₁₋₂₀-Alkyl, besonders bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, isoHexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, insbesondere C₁₋₄-Alkyl, sowie bevorzugt C₄₀₋₂₀₀-Alkyl, wie Polybutyl, Polyisobutyl, Polypropyl, Polyisopropyl und Polyethyl, besonders bevorzugt Polybutyl und Polyisobutyl,
   - Cycloalkyl, wie C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
   - Hydroxyalkyl, wie C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxymethyl-ethyl,
   - Aminoalkyl, wie C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
   - Hydroxyalkylaminoalkyl, wie C₂₋₂₀-Hydroxyalkylaminoalkyl, bevorzugt C₃₋₈-Hydroxyalkylaminoalkyl, wie (2-Hydroxyethylamino)methyl, 2-(2-Hydroxyethylamino)ethyl und 3-(2-Hydroxyethylamino)propyl,
   - Alkoxyalkyl, wie C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂₋₄-Alkoxyalkyl,
   - Dialkylaminoalkyl, wie C₃₋₃₀-Dialkylaminoalkyl, bevorzugt C₃₋₂₀-Dialkylaminoalkyl, besonders bevorzugt C₃₋₁₀-N,N-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-iso-propylamino)ethyl, 3-(N,N-Dimethylamino)propyl, (R⁵)₂N-(CH₂)ₗ,
   - Alkylaminoalkyl, wie C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, 2-Methylaminoethyl, Ethylaminomethyl, 2-Ethylaminoethyl und 2-(isoPropylamino)ethyl, (R⁵)HN-(CH₂)_{q},
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
   - Aralkyl, wie C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenylpropyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - Heteroarylalkyl, wie C₄₋₂₀-Heteroarylalkyl, wie Pyrid-2-yl-methyl, Furan-2-yl-methyl, Pyrrol-3-yl-methyl und Imidazol-2-yl-methyl,
   - Alkylaryl, wie C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - Alkylheteroaryl, wie C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
   - im Fall von ungesättigten Verbindungen der Formel I, Xa und Xb R¹ und R² auch gemeinsam eine -(CH₂)ₗCH₂-X-(CH₂)ₘ- Gruppe, wie -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-,
      oder im Fall von ungesättigten Verbindungen der Formel I R² und R⁴ auch gemeinsam eine -(CH₂)ₗ-X-(CH₂)ₘ- Gruppe, wie -(CH₂)₃-, -(CH₂)₄-, -(CH₂)₅-, -(CH₂)₆-, - (CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-,
R⁵:
   - Wasserstoff (H),
   - Alkyl, besonders C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - Alkylphenyl, besonders C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
X:
   - CH₂, CHR⁵, Sauerstoff (O), Schwefel (S) oder NR⁵, bevorzugt CH₂ und O,
l:
   - eine ganze Zahl von 1 bis 4 (1, 2, 3 oder 4), bevorzugt 1 und 2,
m:
   - eine ganze Zahl von 1 bis 4 (1, 2, 3 oder 4), bevorzugt 1 und 2,

Als im erfindungsgemäßen Verfahren einsetzbare organische Verbindungen mit aliphatisch ungesättigten Gruppen eignen sich praktisch alle Verbindungen mit einer oder auch mehreren aliphatisch (das heißt nicht-aromatisch) ungesättigten CC- oder CN- oder CO-Bindung/en, also aliphatische CC-Doppel- oder CC-Dreifachbindungen (wie olefinische bzw. acetylenische CC-Bindungen), aliphatische CN-Doppel- oder CN-Dreifachbindungen (wie Imin-Gruppen bzw. Nitril-Gruppen) und aliphatische CO-Doppelbindungen (wie Aldehydgruppen oder Ketogruppen).
Die ungesättigten organischen Verbindungen können geradkettig, verzweigt oder zyklisch sein. Hinsichtlich der Kohlenstoffzahl der hydrierbaren Verbindungen gibt es praktisch keine Beschränkungen. Die ungesättigten organischen Verbindungen können ferner Substituenten tragen oder funktionelle Gruppen enthalten, welche sich unter den Bedingungen der Hydrierung inert verhalten, beispielsweise Hydroxy, Alkoxy-, Alkylamino- oder Dialkylaminogruppen, oder gegebenenfalls auch unter den Bedingungen mithydriert werden.
Sollen Verbindungen mit CC- oder CN-Dreifachbindung, wie Alkine bzw. Nitrile, oder Verbindungen mit zwei- oder mehr aliphatisch ungesättigten Gruppen, wie z.B. Diene, Allene, Polyene, Polyine, Alkinene, Enale, Enone, Enimine, hydriert werden, so hat man es von Fall zu Fall über die Steuerung der Reaktionsbedingungen in der Hand, entsprechende mono-, di- oder mehrfach hydrierte Produkte zu erhalten.

Bevorzugt werden beispielsweise die folgenden organischen Verbindungen mit aliphatisch ungesättigten Gruppen hydriert:
Alkene und Alkine, wie Ethylen (zu Ethan), Acetylen (zu Ethylen und/oder Ethan), Propen (zu Propan), Propin (zu Propen und/oder Propan), Propadien (zu Propen und/oder Propan), 1-Buten (zu n-Butan), 2-Buten (zu n-Butan), 1-Butin (zu 1-Buten und/oder n-Butan), 2-Butin (zu 2-Buten und/oder n-Butan), Vinylacetylen (zu 1,3-Butadien), 1,3-Butadien (zu 1-Buten und/oder n-Butan), 1,2-Butadien (zu 1-Buten und/oder n-Butan), Cyclopenten (zu Cyclopentan), 1,3-Cyclopentadien (zu Cyclopenten und/oder Cyclopentan), 1,4-Pentadien (zu 1-Penten und/oder n-Pentan), Cyclohexen (zu Cyclohexan), 1-Vinyl-3-cyclohexen (zu 1-Vinyl-cyclohexan und/oder Ethylcyclohexan), Polyisobuten,
Phenylalkene und -alkine, wie Styrol (zu Ethylbenzol), Phenylacetylen (zu Styrol und/oder Ethylbenzol),
Alkenole und Alkinole, wie 2-Buten-1,4-diol (zu Butan-1,4-diol), 2-Butin-1,4-diol (zu cis-2-Buten-1,4-diol und/oder Butan-1,4-diol),
Alkenale, wie 2-Ethylhexen-2-al (zu 2-Ethylhexanol),
Aldehyde, wie Hydroxypivalinaldehyd (zu Neopentylglykol), 3-Methylpent-1-en-3-al (zu 3-Methylpentanol-3),
Imine, wie Isophoronnitrilimin (IPNI) (zu Isophorondiamin (IPDA)),
Nitrile, wie 3-Dimethylaminopropionitril (zu 3-(Dimethylamino)propylamin),
3-Diethylaminopropionitril (zu 3-Diethylaminopropylamin), 3-Aminopropionitril (zu 1,3-Diaminopropan), Bis(2-Cyanoethyl)amin (zu Dipropylentriamin), N-(2-Cyanoethyl)ethylendiamin (zu N-(3-Aminopropyl)ethylendiamin), N,N'-Di(2-cyanoethyl)ethylendiamin (zu N,N'-Di(3-aminopropyl)ethylendiamin), Adipodinitril (zu Hexamethylendiamin), Isophoronnitril (IPN) (zu Isophorondiamin), 3-Hydroxypropionitril (zu 3-Amino-1-propanol), N-(2-Cyanoethyl)morpholin (zu N-(3-Aminopropyl)morpholin), N-(2-Cyanoethyl)caprolactam (zu 1,8-Diazabicyclo[5.4.0]undecen-7), Isophthalodinitril (zu Metaxylylendiamin), Gemisch aus Adipodinitril und Dimethylamin im Molverhältnis 1 : größer/gleich 2 (zu N,N,N',N'-Tetramethylhexamethylendiamin), Gemisch aus 3-N-Methylaminopropionitril und Monomethylamin im Molverhältnis 1 : größer/gleich 1 (zu N,N'-Dimethylpropylendiamin), Gemisch aus 3-(Dimethylamino)propionitril und 3-(Dimethylamino)propylamin (DMAPA) im Molverhältnis 1 : größer/gleich 1 (zu Bis-[(3-Dimethylamino)propyl]amin (Bis-DMAPA)).

Wie aus den drei letzen Beispielen ersichtlich kann das erfindungsgemäße Verfahren auch zur Herstellung eines sekundären Amins durch Umsetzung von einem Nitril mit einem primären Amin in Gegenwart von Wasserstoff, und zur Herstellung eines tertiären Amins durch Umsetzung von einem Nitril mit einem sekundären Amin in Gegenwart von Wasserstoff, angewendet werden. Bevorzugte Nitrile sind Dinitrile und solche der Formel VI. Bevorzugte eingesetzte Amine sind Monomethylamin, Monoethylamin, Dimethylamin und Diethylamin. Solche Aminsynthesen sind z.B. aus EP-A1-691 157 (BASF AG) bekannt, wobei dem Fachmann weitere Verfahrensdetails aus dieser EP-Schrift geläufig sind.

Das erfindungsgemäße Hydrierverfahren wird im Allgemeinen ähnlich oder genauso wie die bekannten, denselben Zwecken dienenden, heterogen katalysierten Hydrierverfahren kontinuierlich oder diskontinuierlich durchgeführt. Der Katalysator ist bevorzugt als Festbett im Reaktor angeordnet (z.B. Rieselfahrweise oder Sumpffahrweise). Die Ausführungsform als Wirbelbettreaktion mit in auf- und abwirbelnder Bewegung befindlichem Katalysatormaterial ist jedoch ebenfalls möglich.

Das erfindungsgemäße Hydrierverfahren kann als heterogen katalysiertes Gasphasenverfahren, bei dem sich sowohl das Edukt (also die ungesättigte organische Verbindung) wie auch der Hydrierwasserstoff in der Gasphase befinden, oder als heterogen katalysiertes Gas-/Flüssigphasenverfahren, bei dem das Edukt zumindest teilweise in flüssiger Phase und Wasserstoff in der Gasphase und/oder in gelöster Form in der Flüssigphase vorliegt, durchgeführt werden. Die einzustellenden Parameter wie Katalysatorbelastung, Temperatur und Druck werden analog zu denen bekannter Verfahren gewählt. Die Temperatur liegt üblicherweise im Bereich von 20 °C bis 300 °C, insbesondere 40 °C bis 180 °C, und der Absolutdruck im Bereich von 1 bis 320 bar, insbesondere 30 bis 250 bar, (Gas-/Flüssigphasenverfahren), bzw. im Bereich von 1 bis 100 bar, insbesondere 5 bis 80 bar, (Gasphasenverfahren).

Im Gas/Flüssigphasenverfahren kann gegebenenfalls eine Verdünnung der Edukte mit einem geeigneten Lösungsmittel, wie Tetrahydrofuran, Dioxan, N-Methylpyrrolidon oder Ethylenglykoldimethylether, erfolgen.

Die Menge des eingesetzten Wasserstoffs, bezogen auf die Menge des zu hydrierenden Edukts, ist u.a. abhängig von dem Gehalt des Edukts an zu hydrierenden CC-, CN- und CO-Doppel- und CC- und CN-Dreifachbindungen. Im Allgemeinen wird der Wasserstoff in einer Menge im Bereich vom 0,8 bis zum 5-fachen der stöchiometrisch zum vollständigen Wasserstoffumsatz beim Reaktordurchgang erforderlichen Menge zugegeben, vorzugsweise im Bereich vom 0,95 bis 2-fachen dieser Menge. Die Hydrierung von Dreifachbindungen läuft im Normalfall schneller ab als die konjugierter Doppelbindungen, und diese wiederum schneller als die unkonjugierter Doppelbindungen. Dies erlaubt eine entsprechende Steuerung des Verfahrens anhand der zugegebenen Wasserstoffmenge. Es kann auch ein höherer Wasserstoffüberschuss, beispielsweise ein zehnfacher Wasserstoffüberschuss, verwendet werden. Der Wasserstoff kann Inerte enthalten, beispielsweise Edelgase wie Helium, Neon oder Argon, andere inerte Gase wie Stickstoff, Kohlendioxid und/oder niedere Alkane, etwa Methan, Ethan, Propan und/oder Butan. Solche Inertgase im Wasserstoff liegen vorzugsweise in einer Konzentration von weniger als 30 Vol.-% vor. Bevorzugt ist der Wasserstoff frei von Kohlenmonoxid.

Das Verfahren kann kontinuierlich in einem oder in mehreren parallelen oder hintereinandergeschalteten Reaktoren, jeweils im einfachen Durchgang oder in Kreislauffahrweise durchgeführt werden. Bei Durchführung des Verfahrens in der Gas-/Flüssigphase wird der Eduktstrom nach Durchtritt durch einen Reaktor üblicherweise in einem Abscheider von Gasen befreit und ein Teil der erhaltenen Flüssigkeit in den Reaktor zurückgeführt. Das Verhältnis zwischen zurückgeführtem und erstmals in den Reaktor eingespeistem Eduktstrom, das sogenannte Rücklaufverhältnis, wird so eingestellt, dass unter den sonstigen Reaktionsbedingungen, wie Druck, Temperatur, Durchsatz und Wasserstoffmenge, der gewünschte Umsatz erreicht wird.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Aus dem Reaktionsaustrag werden, nachdem dieser zweckmäßigerweise entspannt worden ist, die erhaltenen Hydrierungsprodukte durch z.B. Destillation bzw. Rektifikation, Flüssigextraktion oder Kristallisation gereinigt. Überschüssiger Wasserstoff wird vorteilhaft wieder in die Reaktionszone zurückgeführt. Das gleiche gilt für eventuell nicht vollständig umgesetztes Edukt.

Die unter Anwendung des erfindungsgemäßen Verfahrens hergestellten Produkte der katalytischen Hydrierung sind z.B. wichtige Zwischenprodukte der chemischen Industrie, wie z.B. das so aus 2-Buten-1,4-diol und 2-Butin-1,4-diol hergestellte 1,4-Butandiol.

### Beispiele

### Herstellung von Katalysatoren

### Katalysator A (erfindungsgemäß):

Katalysator A wurde durch Auffällung der Komponenten Cu, Co, Ni auf monoklines Zirkoniumdioxid, das im Fällkessel vorgelegt wurde, wie folgt hergestellt:

In einem Rührkessel aus Glas wurde eine Suspension aus 155 g monoklinem Zirkoniumdioxid-Pulver (BET = 105 m² g⁻¹) in 2 l Wasser vorgelegt und unter Rühren auf 70 °C erhitzt. Innerhalb von 30 Min. wurde dann eine Lösung aus 190,1 g Cu(NO₃)₂ x 2,5 H₂O, 561,9 g Ni(NO₃)₂ x 6 H₂O und 543,7 g Co(NO₃)₂ x 6 H₂O in 2,8 I Wasser zugetropft. Durch gleichzeitiges Zutropfen einer 20 %-igen Sodalösung (700 g Na₂CO₃ in 2,8 I Wasser) wurde der pH bei konstant 6,0 gehalten. Nach der Zugabe der Lösungen wurde für 1 h bei 70 °C nachgerührt und schließlich durch Zugabe von Sodalösung der pH-Wert auf 7,4 erhöht. Die Suspension wurde auf eine Nutsche gepumpt und mit 100 I Wasser gewaschen. Der Filterkuchen wurde für 16 h bei 200 °C im Trockenschrank getrocknet, anschließend auf Korngrößen < 1,6 mm zerkleinert und für 2 h bei 400 °C in einem Drehrohrofen im Luftstrom (150 l/h) calciniert.

Das so erhaltene Katalysatorpulver hatte die Zusammensetzung:
28 Gew.-% Ni, berechnet als NiO,
28 Gew.-% Co, berechnet als CoO,
11 Gew.-% Cu, berechnet als CuO, und
33 Gew.-% Zr, berechnet als ZrO₂.

Das Pulver wurde mit 3 Gew.-% Graphit versetzt, kompaktiert, wieder auf < 1,6 mm zerkleinert, und schließlich zu 4,75 x 3 mm-Tabletten gepresst. Die Tabletten wurden für 2 h bei 400 °C im Muffelofen an Luft nachcalciniert. In einer Reduktionskolonne wurden die Tabletten dann in einem Wasserstoff/Stickstoffstrom reduziert, zunächst 6 h bei 150 °C, dann 6 h bei 280 °C. Nach Abkühlung auf Raumtemperatur wurden die Tabletten dann in einem verdünnten Luftstrom passiviert.

### Katalysator B (Vergleichskatalysator):

Der Vergleichskatalysator wurde wie der erfindungsgemäße Katalysator A hergestellt, mit dem Unterschied, dass kein monoklines Zirkoniumdioxid vorgelegt wurde. Anstelle dessen wurden zu der Cu-, Co- und Ni-Nitrathaltigen Metallsalzlösung 775 g einer Zirkoniumacetatlösung, enthaltend 20 Gew.-% ber. ZrO₂, (bezogen auf das Gewicht der Zirkoniumacetatlösung) zugegeben (Mischfällung). Die weitere Herstellung erfolgte analog Katalysator A. Das analog erhaltene Katalysatorpulver hatte die gleiche Zusammensetzung wie beim Katalysator A beschrieben.

### Beispiel 1:

Zur Testung der mechanischen Stabilität der Katalysatoren A und B unter hydrierenden Reaktionsbedingungen wurden unter Reaktionsbedingungen der hydrierenden Aminierung von hydroformylierten Polyisobuten (PIB-Oxo; Molmasse: 1000) zum primären Amin PIBA nach dem Reaktionsschema 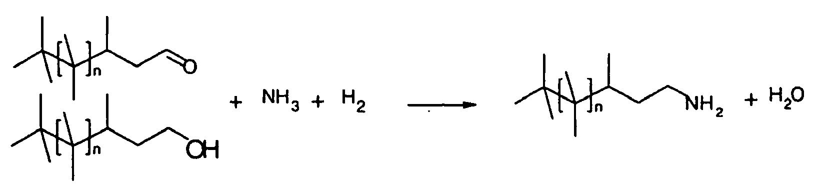 die Katalysatoren in einem Autoklaven einem sog. Kochtest (Autoklaventest) unterzogen, bei dem die Reaktionsbedingungen wie folgt nachgestellt wurden:
20 g Katalysator wurden in einen Drahtkorb in einem Rührautoklaven gegeben. Dazu wurden 150 ml einer PIBA/Mihagol (50/50) Lösung gegeben, so dass die Katalysatortabletten gut mit Flüssigkeit überdeckt waren. Der Autoklav wurde verschlossen, mit Stickstoff gespült, der Rührer mit 700 U/min betrieben, 50 bar H₂ aufgepresst und auf 200 °C aufgeheizt. Der Druck wurde dann durch Zupressen von H₂ auf 200 bar eingestellt. Unter diesen Bedingungen wurde der Katalysator für unterschiedliche Zeiträume behandelt. Anschließend wurde abgekühlt, vorsichtig entspannt und die mechanische Stabilität des Katalysators durch Messung der Seitendruckfestigkeit (SDF) bestimmt.

Hierzu wurde die Katalysatortablette zwischen zwei parallelen Platten auf der Mantelseite mit zunehmender Kraft belastet, bis Bruch eintrat. Die beim Bruch registrierte Kraft ist die Seitendruckfestigkeit. Die Bestimmung erfolgte auf einem Prüfgerät der Firma Zwick, Ulm, mit festsitzendem Drehteller und frei beweglichem, vertikalem Stempel, der den Formkörper gegen den festsitzenden Drehteller drückte. Der frei bewegliche Stempel war mit einer Druckmessdose zur Aufnahme der Kraft verbunden. Das Gerät wurde durch einen Computer gesteuert, welcher die Messwerte registrierte und auswertete. Aus einer gut durchmischten Katalysatorprobe wurden 25 einwandfreie (d.h. rissefrei und ohne abgestoßenen Kanten) Tabletten entnommen, deren Seitendruckfestigkeit ermittelt und anschließend gemittelt.

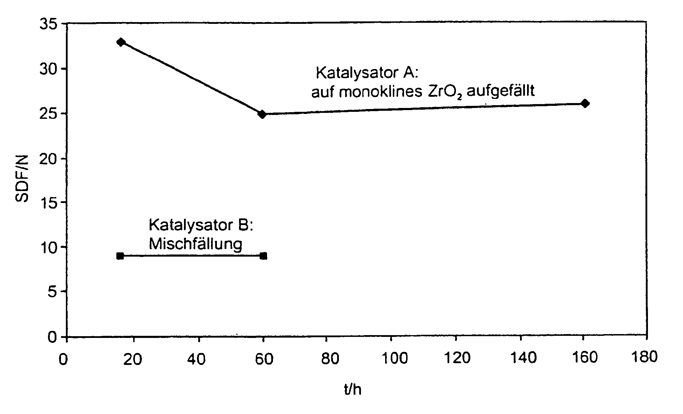

In der beiliegenden Abbildung 1 sind die Seitendruckfestigkeiten (Einheit: Newton, N) der beiden Katalysatoren A und B über die Dauer des Autoklaventests (in Stunden) aufgetragen:

Bei dem durch Mischfällung hergestellten Kontakt B zeigte das Röntgendiffraktogramm nach erfolgtem Kochtest, dass sich die zunächst röntgenamorphe ZrO₂-Phase in tetragonales und monoklines ZrO₂ umgewandelt hatte. Bei Katalysator A wurde keine Umkristallisation (= Änderung der Modifikation) festgestellt.

## Patentansprüche

1. Verfahren zur katalytischen Hydrierung einer aliphatisch ungesättigten Gruppe in einer organischen Verbindung, **dadurch gekennzeichnet, dass** man einen Katalysator einsetzt, bei dessen Herstellung eine Fällung von katalytisch aktiven Komponenten auf monoklines, tetragonales oder kubisches Zirkoniumdioxid erfolgte, und dass die katalytisch aktive Masse des Katalysators vor der Behandlung mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO, und
14 bis 70 Gew.-% sauerstoffhaltige Verbindungen des Nickels, berechnet als NiO, enthält.

2. Verfahren nach dem vorhergehenden Anspruch, **dadurch gekennzeichnet, dass** es sich bei den aufgefällten katalytisch aktiven Komponenten um Metallsalze handelt, wobei das Metall aus den Nebengruppen VIII und IB des Periodensystems ausgewählt ist.

3. Verfahren nach dem vorhergehenden Anspruch, **dadurch gekennzeichnet, dass** es sich bei den Metallsalzen um in Wasser schwer- oder unlösliche, basische Salze handelt.

4. Verfahren nach einem der beiden vorhergehenden Ansprüche, **dadurch gekennzeichnet, dass** es sich bei den Salzen um Oxide, Oxidhydrate, Hydroxide, Carbonate und/oder Hydrogencarbonate handelt.

5. Verfahren nach einem der Ansprüche 2 bis 4, **dadurch gekennzeichnet, dass** das Metall aus der Gruppe Fe, Co, Ni, Ru, Rh, Pd, Pt und Cu ausgewählt ist.

6. Verfahren nach einem der Ansprüche 2 bis 4, **dadurch gekennzeichnet, dass** das Metall aus der Gruppe Cu, Ni und Co ausgewählt ist.

7. Verfahren nach einem der vorhergehenden Ansprüche, **dadurch gekennzeichnet, dass** die katalytisch aktive Masse des Katalysators vor der Behandlung mit Wasserstoff
20 bis 65 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, und
15 bis 50 Gew.-% sauerstoffhaltige Verbindungen des Kobalts, berechnet als CoO, enthält.

8. Verfahren nach einem der vorhergehenden Ansprüche, **dadurch gekennzeichnet, dass** das Molverhältnis von Nickel zu Kupfer größer 1 ist.

9. Verfahren nach einem der vorhergehenden Ansprüche, **dadurch gekennzeichnet, dass** das monokline, tetragonale oder kubische Zirkoniumdioxid ein oder mehrere Metalloxide der Nebengruppe IIIB oder der Hauptgruppe IIA des Periodensystems enthält.

10. Verfahren nach einem der vorhergehenden Ansprüche, **dadurch gekennzeichnet, dass** man die Hydrierung bei Temperaturen von 20 bis 300 °C durchführt.

11. Verfahren nach einem der vorhergehenden Ansprüche, **dadurch gekennzeichnet, dass** man die Hydrierung in der Gas-/Flüssigphase bei Absolutdrücken von 1 bis 320 bar oder in der Gasphase bei Drücken von 1 bis 100 bar durchführt.

12. Verfahren nach einem der vorhergehenden Ansprüche, wobei es sich bei der ungesättigten Gruppe um eine aliphatische CC-Doppel- oder CN-Doppelbindung handelt.

13. Verfahren nach einem der Ansprüche 1 bis 11, wobei es sich bei der ungesättigten Gruppe um eine aliphatische CC-Dreifach- oder CN-Dreifachbindung handelt.

14. Verfahren nach einem der Ansprüche 1 bis 11, wobei es sich bei der aliphatisch ungesättigten Gruppe um eine Aldehyd- oder Ketogruppe handelt.

15. Verfahren nach einem der Ansprüche 1 bis 11 zur Herstellung eines sekundären Amins, wobei es sich bei der aliphatisch ungesättigten Gruppe um eine Nitrilgruppe handelt und eine Umsetzung mit einem primären Amin erfolgt.

16. Verfahren nach einem der Ansprüche 1 bis 11 zur Herstellung eines tertiären Amins, wobei es sich bei der aliphatisch ungesättigten Gruppe um eine Nitrilgruppe handelt und eine Umsetzung mit einem sekundären Amin erfolgt.

17. Verwendung eines Katalysators gemäß einem der Ansprüche 1 bis 9 zur Hydrierung einer aliphatisch ungesättigten Gruppe in einer organischen Verbindung.

18. Verwendung gemäß Anspruch 17 zur Hydrierung einer aliphatischen CC-Doppel- oder CN-Doppelbindung, aliphatischen CC-Dreifach- oder CN-Dreifachbindung, oder Aldehyd- oder Ketogruppe.

## Claims

1. A process for the catalytic hydrogenation of an aliphatically unsaturated group in an organic compound, wherein a catalyst whose preparation has involved precipitation of catalytically active components onto monoclinic, tetragonal or cubic zirconium dioxide is used and the catalytically active composition of the catalyst before treatment with hydrogen comprises from 20 to 85% by weight of oxygen-containing compounds of zirconium, calculated as ZrO₂, from 1 to 30% by weight of oxygen-containing compounds of copper, calculated as CuO, and from a to 70% by weight of oxygen-containing compounds of nickel, calculated as NiO.

2. The process according to the preceding claim, wherein the catalytically active components precipitated are salts of a metal selected from transition groups VIII and IB of the Periodic Table.

3. The process according to the preceding claims, wherein the metal salts are basic salts which are sparingly soluble or insoluble in water.

4. The process according to either of the two preceding claims, wherein the salts are oxides, hydrated oxides, hydroxides, carbonates and/or hydrogencarbonates.

5. The process according to any of claims 2 to 4, wherein the metal is selected from the group consisting of Fe, Co, Ni, Ru, Rh, Pd, Pt and Cu.

6. The process according to any of claims 2 to 4, wherein the metal is selected from the group consisting of Cu, Ni and Co.

7. The process according to any of the preceding claims, wherein the catalytically active composition of the catalyst before treatment with hydrogen comprises from 20 to 65% by weight of oxygen-containing compounds of zirconium, calculated as ZrO₂, from 1 to 30% by weight of oxygen-containing compounds of copper, calculated as CuO, from 15 to 50% by weight of oxygen-containing compounds of nickel, calculated as NiO, and from 15 to 50% by weight of oxygen-containing compounds of cobalt, calculated as CoO.

8. The process according to any of the preceding claims, wherein the molar ratio of nickel to copper is greater than 1.

9. The process according to any of the preceding claims, wherein the monoclinic, tetragonal or cubic zirconium dioxide contains one or more oxides of metals of transition groups IIIB or main group IIA of the Periodic Table.

10. The process according to any of the preceding claims, wherein the hydrogenation is carried out at from 20 to 300°C.

11. The process according to any of the preceding claim, wherein the hydrogenation is carried out in the gas/liquid phase at absolute pressures of from 1 to 320 bar or in the gas phase at pressures of from 1 to 100 bar.

12. The process according to any of the preceding claims, wherein the unsaturated group is an aliphatic CC double bond or CON double bond.

13. The process according to any of claims 1 to 11, wherein the unsaturated group is an aliphatic CC triple bond or CN triple bond.

14. The process according to any of claims 1 to 11, wherein the aliphatically unsaturated group is an aldehyde group or keto group.

15. The process according two any of claims 1 to 11 for preparing a secondary amine, wherein the aliphatically unsaturated group is a nitrile group and a reaction with a primary amine is carried out.

16. The process according to any of claims 1 to 11 for preparing a tertiary amine, wherein the aliphatically unsaturated group is a nitrile group and a reaction with a secondary amine is carried out.

17. The use of a catalyst according to any of claims 1 to 9 for the hydrogenation of an aliphatically unsaturated group in an organic compound.

18. The use according to claim 17 for the hydrogenation of an aliphatic CC double bond or CN double bond, an aliphatic CC triple bond or CN triple bond or an aldehyde group or keto group.

## Revendications

1. Procédé d'hydrogénation catalytique d'un groupe aliphatique insaturé dans un composé organique, **caractérisé en ce que** l'on met en oeuvre un catalyseur dans la préparation duquel a été opérée une précipitation de composants à activité catalytique sur du dioxyde de zirconium monoclinique, tetragonal ou cubique, et que la masse à activité catalytique du catalyseur contient avant le traitement par l'hydrogène :
de 20 à 85 % en poids de composés oxygénés du zirconium, calculé en ZrO₂,
de 1 à 30 % en poids de composés oxygénés du cuivre, calculé en CuO, et
de 14 à 70 % en poids de composés oxygénés du nickel, calculé en NiO.

2. Procédé suivant la revendication qui précède, **caractérisé en ce que** les composants à activité catalytique précipités sont des sels métalliques, le métal étant choisi dans les sous-groupes VIII et IB du système périodique.

3. Procédé suivant la revendication qui précède, **caractérisé en ce que** les sels métalliques sont des sels basiques peu solubles ou insolubles dans l'eau.

4. Procédé suivant l'une des deux revendications qui précèdent, **caractérisé en ce que** les sels sont des oxydes, des oxyhydrates, des hydroxydes, des carbonates et/ou des hydrogénocarbonates.

5. Procédé suivant l'une quelconque des revendications 2 à 4, **caractérisé en ce que** le métal est choisi dans le groupe formé par Fe, Co, Ni, Ru, Rh, Pd, Pt et Cu.

6. Procédé suivant l'une quelconque des revendications 2 à 4, **caractérisé en ce que** le métal est choisi dans le groupe formé par Cu, Ni et Co.

7. Procédé suivant l'une quelconque des revendication qui précèdent, **caractérisé en ce que** la masse à activité catalytique du catalyseur contient avant le traitement par l'hydrogène :
de 20 à 65 % en poids de composés oxygénées du zirconium, calculé en ZrO₂,
de 1 à 30 % en poids de composés oxygénés du cuivre, calculé en CuO,
de 15 à 50 % en poids de composés oxygénés du nickel, calculé en NiO, et
de 15 à 50 % en poids de composés oxygénées du cobalt, calculé en CoO.

8. Procédé suivant l'une quelconque des revendications qui précèdent, **caractérisé en ce que** la proportion molaire du nickel au cuivre est de plus de 1.

9. Procédé suivant l'une quelconque des revendications qui précèdent, **caractérisé en ce que** le dioxyde de zirconium monoclinique, tétragonal ou cubique contient un ou plusieurs oxydes de métaux du sous-groupe IIIB ou du groupe principal IIA du système périodique.

10. Procédé suivant l'une quelconque des revendications qui précèdent, **caractérisé en ce que** l'on entreprend l'hydrogénation à des températures de 20 à 300°C.

11. Procédé suivant l'une quelconque des revendications qui précédent, **caractérisé en ce que** l'son entreprend l'hydrogénation dans la phase gaz/liquide à des paressions absolues de 1 à 320 bars ou dans la phase gazeuse à des paressions de 1 à 100 bars.

12. Procédé suivant l'une quelconque des revendication qui précèdent dans lequel le groupe insaturé est une double liaison CC ou une double liaison CN aliphatique.

13. Procédé suivant l'une quelconque des revendications 1 à 11 dans lequel le groupe insaturé est une triple liaison CC ou une triple liaison CN aliphatique.

14. Procédé suivant l'une quelconque des revendications 1 à 11 dans lequel le groupe aliphatique insaturé est un groupe aldéhyde ou cétone.

15. Procédé suivant l'une quelconque des revendications 1 à 11 pour la préparation d'une amine secondaire, où le groupe aliphatique insaturé est un groupe nitrile et il se produit une réaction avec une amine frimaire.

16. Procédé suivant l'une quelconque des revendications 1 à 11 pour la préparation d'une amine tertiaire, où le groupe aliphatique insaturé est un groupe nitrile et il se produit une réaction avec une amine secondaire.

17. Utilisation d'un catalyseur suivant l'une quelconque des revendications 1 à 9 pour l'hydrogénation d'un groupe aliphatique insaturé dans un composé organique.

18. Utilisation suivant la revendication 17 pour l'hydrogénation d'une double liaison CC ou d'une double liaison CN aliphatique, d'une triple liaison CC ou d'une triple liaison CN aliphatique, ou d'un groupe aldéhyde ou cétone.
